# EUROPÄISCHE PATENTSCHRIFT

(11) **EP 0 916 950 B1**
(45) Veröffentlichungstag und Bekanntmachung des Hinweises auf die Patenterteilung: **21.11.2001**
(21) Anmeldenummer: 98113954.6
(22) Anmeldetag: 25.07.1998
(51) Int. Cl.: G01N 33/543, G01N 33/558, G01N 33/58

(54) **Immunassayvorrichtung zum Nachweis von Nikotin oder Cotinin**
Immunoassay device for determining nicotine or cotinine
Dispositif d'essais immunologiques pour la détection de nicotine ou cotinine

(30) Priorität: 17.11.1997 DE 19750869
(43) Veröffentlichungstag der Anmeldung: 19.05.1999
(73) Patentinhaber: Unlimited Pharmaceutical Deutschland GmbH, 22926 Ahrensburg (DE)
(72) Erfinder: Engel, Matthias, 22926 Ahrensburg (DE); Schwarz, Ulrich, Dr., D-23858 Reinfeld/Holstein (DE)
(74) Vertreter: Siewers, Gescha, Dr.

(56) Entgegenhaltungen:
- EP-A2- 0 194 158

## Beschreibung

Die Erfindung betrifft eine Immunassayvorrichtung zum Nachweis von Nikotin oder Cotinin.

Nikotin ist das Hauptalkaloid des Tabaks, wobei der Nikotingehalt der Handelstabake zwischen etwa 0,3 bis 3 und bei schweren Sorten bis zu 7% betragen kann. Nikotin wird allerdings im Körper relativ schnell abgebaut; der Hauptmetabolit des Nikotins ist Cotinin, das dem Nikotin entsprechende 2-Pyrrolidinon. Während Nikotin eine biologische Halbwertszeit menschlichen Blut von ungefähr einer halben Stunde hat, hat Cotinin, das als Nebenalkaloid auch im Tabak vorkommt, eine biologische Halbwertszeit von etwa 30 bis 40 Stunden und tritt in Blut, Serum oder Urin von Rauchern in wesentlich höheren Konzentrationen als Nikotin auf. Der Nachweis von Cotinin wird daher als verläßlicher Indikator für Rauchen oder Nichtrauchen angesehen.

Da einerseits Rauchen in bestimmten Arbeitsumgebungen wie beispielsweise in der chemischen oder Ölindustrie wegen der damit verbundenen Brand- oder Explosionsgefahr ein beträchtliches Risiko darstellen kann und andererseits zwar nicht den im Tabak enthaltenen Alkaloiden, aber anderen beim Rauchen entstehenden Begleitprodukten auch ein beträchtliches gesundheitliches Risiko nachgesagt wird, besteht schon seit langem nicht nur bei der verarbeitenden Industrie, sondern beispielsweise auch bei Versicherungsgesellschaften ein Wunsch nach einem schnell durchzuführenden und sicheren Test zur Überprüfung, ob es sich bei Personen, die sich als Nichtraucher ausgeben, tatsächlich auch um Nichtraucher handelt. Schnell und einfach durchführbare Tests auf Nikotin oder Cotinin sind aber bisher nicht vorhanden.

Es ist allerdings bereits bekannt, daß sich Cotinin als Hauptmetabolit des Nikotin zum Nachweis des Rauchens mit Hilfe von Immunassays eignet; so sind von van Vunakis, H. et al. in Am. J. Obstet. Gynecol. 120, 64 ff (1974) ein Radioimmunassay zum Nachweis von Nikotin und Cotinin in der Amnionsflüssigkeit von schwangeren Raucherinnen und von Bjercke, R. J. et al. ELISA-Test zum Nachweis von Antikörpern gegen Nikotin und Cotinin in Journal of Immunological Methods, 90 (1986) 203 pp. beschrieben worden. Zwar sind Radioimmunassays und ELISA-Tests relativ sichere Nachweismethoden für Nikotin oder Cotinin, aber sie sind nur von Fachleuten durchzuführen und zeitaufwendig, also als sicherer und bequem handzuhabender Schnelltest völlig ungeeignet.

Überraschenderweise wurde nunmehr festgestellt, daß ein solcher Schnelltest auf Nikotin oder Cotinin sich auch auf Basis eines Immunassay durchführen läßt, wenn eine Immunassayvorrichtung gemäß Hauptanspruch eingesetzt wird.

Immunassays sind hochsensitive Bestimmungsverfahren, die auf der Spezifität immunologischer Reaktionen, und zwar in erster Linie von Antigen- und Antikörper-Reaktionen beruhen. Für die qualitative oder quantitative Auswertung ist die Markierung eines der Reaktionspartner mit einer gut nachweisbaren und meßbaren Indikatorsubstanz wie beispielsweise radioaktive Isotope, Enzyme, Farbstoffe und Fluoreszenzfarbstoffe notwendig. Meist handelt es sich um Festphasentests, in denen der an der festen Phase gebundene Marker gemessen wird. Als Festphase dienen Mikrotiterplatten wie im Falle der ELISA-Tests, oder feste Träger, von denen sich insbesondere Nitrocellulose bewährt hat. Festphasentests sind so aufgebaut, daß ein fester Träger in verschiedene Sektionen unterteilt ist, wobei das zu untersuchende flüssige wäßrige Medium auf den Anfangsteil des Trägers aufgebracht wird und dann die Flüssigkeit aufgrund der Kapillarität des Trägers diesen vollständig durchziehen kann, wobei überschüssige Flüssigkeit abgespült oder in einer sogenannten Flüssigkeitsfalle abgefangen wird. Der feste Träger ist innerhalb einer ersten Sektion entweder mit markiertem Antigen oder markiertem Antikörper versehen, so daß bei Zusatz der in Lösung befindlichen nachzuweisenden Substanz eine Antigen-Antikörper-Reaktion eintritt, wobei der Marker entweder an das Antigen oder an den Antikörper gekoppelt sein kann. Das Antigen-Antikörper-Reaktionsprodukt migriert dann mit der Flüssigkeit in eine zweite Sektion, die entweder ein immobilisiertes Antigen oder einen immobilisierten Antikörper aufweist, mit dem das Produkt aus nachzuweisender Substanz und Antigen oder Antikörper in einer Kompetitivreaktion sich umsetzt bzw., sofern es sich um einen Sandwichtest handelt, doppelt umgesetzt wird. Dadurch tritt eine Konzentration des Markers in diesem Bereich ein, die dann qualitativ oder quantitativ mit einem entsprechenden Meßgerät oder, soweit im sichtbaren Bereich adsorbierende Farbstoffe als Marker eingesetzt werden, mit dem Auge wahrgenommen werden kann. Im Überschuß eingesetzte und daher nicht verbrauchte markierte Antigene oder Antikörper können ggf. in eine dritte Sektion weitermigrieren, in der sie dann beispielsweise durch Antigene oder Antikörper mit anderer Spezifität gebunden und somit als Kontrolle eingesetzt werden können.

Festphasenimmunassays in Kompetitiv- oder Sandwichtechnik sind dem Fachmann bekannt und brauchen daher nicht näher erläutert zu werden. Solche Tests sind beispielsweise in der EP-A1 0 284 232 oder in der EP-B1 0 291 194 im Detail beschrieben.

Die erfindungsgemäße Vorrichtung besteht aus einem sogenannten "Dipstick", also einem porösem Träger, in dem das Kopplungsprodukt aus Nachweisreagenz und nachzuweisender Substanz aufgrund der Kapillarität migrationsfähig ist und der allgemein zur Verbesserung seiner mechanischen Eigenschaften seinerseits auf einem Träger wie beispielsweise einer Kunststoffplatte, befestigt ist. Der Träger, d.h. also das Festphasenmaterial, kann aus unterschiedlichen porösen und damit eine Kapillarität bedingenden Materialien bestehen wie Cellulose, Polyamid, vorbehandelte Papiere usw., aber vorzugsweise wird Nitrocellulose verwendet. Der Träger weist eine erste Sektion auf, die ein markiertes Nachweisreagenz für die nachzuweisende Substanz trägt, und zwar je nach Anordnung des Tests, entweder einen markierten Antikörper oder ein markiertes Antigen. Die Markierung erfolgt mit teilchenförmigen Partikeln, die vorzugsweise entweder kleine gefärbte Kunststoffpartikel, insbesondere aus Polystyrol, darstellen oder Metallsole, und zwar insbesondere Goldsol, da dieses auch in großer Verdünnung eine mit dem bloßem Augen gut sichtbare blaustichigrote Färbung aufweist. Beim Befeuchten dieser ersten Sektion mit der in Lösung befindlichen nachzuweisenden Substanz entsteht ein Kopplungsprodukt aus markiertem Nachweisreagenz und der zu analysierenden Verbindung, wobei dieses Produkt im feuchten Zustand des Trägers aus der ersten Sektion in die zweite Sektion migrieren kann. In der zweiten Sektion befindet sich ein immobilisiertes Bindereagenz, das wiederum, je nach Auslegung des Tests, ein nicht-markierter Antikörper oder ein nicht-markiertes Antigen sein kann.

Falls erwünscht, kann die Vorrichtung noch eine dritte Sektion, nämlich eine Kontrollzone enthalten, an der abgelesen werden kann, ob der Vorgang korrekt abgelaufen ist. Die nicht durch die Kopplung mit dem Bindungsreagenz verbrauchten markierten Antigene oder Antikörper können dann beispielsweise in dieser Kontrollzone mit einem immobilisierten Antigen oder Antikörper mit anderer Spezifität umgesetzt werden oder die Kontrollzone kann eine Substanz enthalten, die beispielsweise mit dem wäßrigen Träger durch Farbänderung reagiert, so daß auf die eine oder andere Art angezeigt wird, daß die wäßrige Lösung, die in der ersten Sektion aufgetragen wurde, die Stelle des Kontrollstreifens tatsächlich erreicht hat und somit die Reaktion als beendet anzusehen ist.

Vorzugsweise wird erfindungsgemäß die Reaktion so durchgeführt, daß in der ersten Sektion ein migrationsfähiger Antikörper und in der zweiten Sektion ein immobilisiertes Antigen eingesetzt werden, wobei beispielsweise Antigene und Antikörper eingesetzt werden können, wie sie in der bereits zitierten Arbeit von Bjercke, R. J. et al. ausführlich beschrieben werden. Die Antikörper werden zur Verwendung mit einem Marker, insbesondere mit Goldsol konjugiert.

Falls eine dritte Sektion mit einer Kontrollmöglichkeit erwünscht ist, kann in dieser dritten Sektion beispielsweise ein im Handel erhältliches "Antimaus"-Immunglobulin aus Kaninchen eingesetzt werden, das dort immobilisiert wird, wenn in der ersten Sektion mit Antikörpern aus der Maus gearbeitet wird.

Um die Vorrichtung bzw. den Dipstrip vor Umwelteinflüssen wie Feuchtigkeit, Staub usw. zu schützen, befindet dieser sich vorzugsweise in einer Hülle, die den gesamten Streifen bis auf einen Teil der ersten Sektion umschließt. Die Hülle besteht vorzugsweise aus Kunststoff, der wasserabweisend und staubdicht ist. In einer weiteren bevorzugten Ausführungsform ist die Hülle mindestens zweiteilig, so daß der aus der Hülle herausragende Teil des Teststreifens ebenfalls durch einen abziehbaren Teil der Hülle abgedeckt werden kann. Der aus der Hülle oder einem Teil der Hülle herausragende Teil des Teststreifens mit der ersten Sektion wird so gewählt, daß dieser Teil mit der zu untersuchenden Körperflüssigkeit, also insbesondere Urin, getränkt werden kann und dann eine gleichmäßige Migration der Tränkungsflüssigkeit durch den Gesamtstreifen erfolgt.

Die Erfindung wird nunmehr im folgenden anhand der Zeichnungen näher erläutert.
- Fig. 1: zeigt in schematischer Darstellung eine erfindungsgemäße Vorrichtung, bei der als markiertes Nachweisreagenz ein Farbstoff tragender Antikörper und als Bindereagenz ein Antigen eingesetzt werden und
- Fig. 2: zeigt in schematischer Darstellung den Fall, in dem das markierte Nachweisreagenz ein Antigen ist und das Bindereagenz ein Antikörper.

Auf einem trockenen porösen Teststreifen aus beispielsweise Nitrocellulose sind drei Sektionen (1, 2, 3) unterteilt, in denen wäßrige Flüssigkeit durch Kapillarität migrieren kann. Die Überlappungsgrenzen zwischen diesen Sektionen sind gestrichelt dargestellt.

Bei Verwendung von einem markierten Antikörper als Nachweisreagenz wandert dieser nach Umsetzung mit der zu analysierenden Substanz über die Sektionsgrenze von Sektion 1 in Sektion 2, bis die Linie erreicht ist, auf der sich als Bindereagenz immobilisiertes Antigen befindet. Diese Testlinie ist mit 4 bezeichnet. Überschüssiges Nachweisreagenz migriert dann weiter über die Grenze zwischen einer zweiten und dritten Sektion (2, 3) in die Kontrollsektion 3 und trifft dort auf eine Linie einer immobilisierten Nachweissubstanz anderer Spezifität (5). Der Teststreifen ist bis auf einen Teil der ersten Sektion von einem Gehäuse (6) umschlossen.

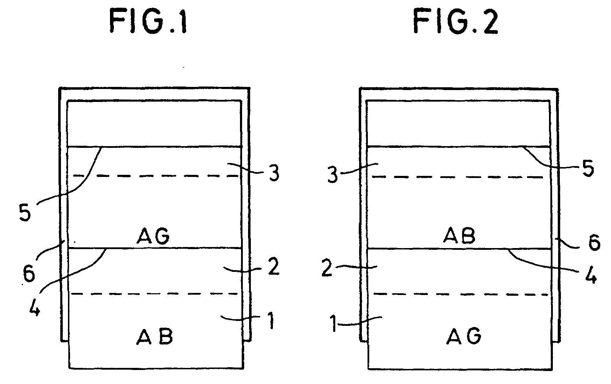

Die Reaktion kann aber auch so durchgeführt werden, daß, wie in Fig. 2 dargestellt, die Sektion 1 als markiertes Nachweisreagenz ein Antigen enthält, das nach Umsetzung mit der nachzuweisenden Substanz aus der Analysenflüssigkeit über die Grenze zwischen den Sektionen 1 und 2 bis zu einem immobilisierten Antikörper auf der Linie (4) migriert und von dort aus weiter über die Grenze zwischen den Sektionen (2, 3) bis zu einem weiteren immobilisierten Nachweisreagenz mit anderer Spezifität (5).

## Patentansprüche

1. Immunassayvorrichtung, bestehend aus einem trockenen porösen Träger mit mindestens zwei, miteinander im feuchten Zustand durch Kapillarität verbundenen Sektionen (1, 2), wobei die erste Sektion (1) ein markiertes Nachweisreagenz für die nachzuweisende Substanz in Form eines entsprechenden Antikörpers oder entsprechenden Antigens und die zweite Sektion (2) ein immobilisiertes Bindereagenz für die nachzuweisende Substanz in Form eines Antigens oder Antikörpers aufweisen und wobei das Reaktionsprodukt aus nachzuweisender Substanz und markiertem Nachweisreagenz aufgrund von Kapillarität von der ersten in die zweite Sektion im feuchten Zustand migriert, **dadurch gekennzeichnet, daß** es sich bei dem markierten Nachweisreagenz um ein Farbstoff tragendes Reagenz für Nikotin oder Cotinin handelt.

2. Immunassayvorrichtung nach Anspruch 1, **dadurch gekennzeichnet, daß** das immobilisierte Bindereagenz ein Nikotin- oder Cotininantigen ist.

3. Immunassayvorrichtung nach Anspruch 1, **dadurch gekennzeichnet, daß** das immobilisierte Bindereagenz ein Nikotin- oder Cotininantikörper ist.

4. Immunassayvorrichtung nach Anspruch 1 bis 3, **dadurch gekennzeichnet, daß** das migrierende Reaktionsprodukt aus nachzuweisender Substanz und markiertem Nachweisreagenz mit gefärbten Kunststoffpartikeln oder Metallsolen konjugiert ist.

5. Immunassayvorrichtung nach Anspruch 4, **dadurch gekennzeichnet, daß** das Konjugat ein Goldsol enthält.

6. Immunassayvorrichtung nach Anspruch 1 bis 5, **dadurch gekennzeichnet, daß** sich zum Nachweis der fehlerfreien Durchführung des Tests an die zweite Sektion (2) eine Kontrollsektion (3) anschließt.

7. Immunassayvorrichtung nach Anspruch 6, **dadurch gekennzeichnet, daß** in der Kontrollsektion (3) eine Umsetzung des markierten Antigens oder Antikörpers oder des wäßrigen Trägers zu einem farbigen Reaktionsprodukt erfolgt.

8. Immunassayvorrichtung nach Anspruch 1 bis 7, **dadurch gekennzeichnet, daß** die Vorrichtung sich in einer gegen Umwelteinflüsse schützenden Hülle (6) befindet.

9. Immunassayvorrichtung nach Anspruch 8, **dadurch gekennzeichnet, daß** die Hülle aus Kunststoff besteht.

10. Immunassayvorrichtung nach Anspruch 9, **dadurch gekennzeichnet, daß** die Hülle (6) zweiteilig ist.

## Claims

1. Immunoassay apparatus, consisting of a dry, porous carrier with at least two sections (1, 2) interconnected in the wet state by capillarity, in which the first section (1) has a labelled detection reagent for the substance to be detected in the form of a corresponding antibody or corresponding antigen and the second section (2) has an immobilised reagent for binding the substance to be detected in the form of an antigen or antibody, and in which the reaction product of the substance to be detected and the labelled reagent migrates in the wet state from the first to the second section due to capillarity, **characterised in that** the labelled detection reagent is a colorant-bearing reagent for nicotine or cotinine.

2. Immunoassay apparatus according to Claim 1, **characterised in that** the immobilised binding reagent is a nicotine antigen or cotinine antigen.

3. Immunoassay apparatus according to Claim 1, **characterised in that** the immobilised binding reagent is a nicotine antibody or cotinine antibody.

4. Immunoassay apparatus according to Claim I to 3, **characterised in that** the migrating reaction product of the substance to be detected and the labelled detection reagent is conjugated with coloured plastics materials or metal sols.

5. Immunoassay apparatus according to Claim 4, **characterised in that** the conjugate contains a gold sol.

6. Immunoassay apparatus according to Claim 1 to 5, **characterised in that**, to detect error-free performance of the test, a control section (3) is joined to the second section (2).

7. Immunoassay apparatus according to Claim 6, **characterised in that** the labelled antigen or antibody or the aqueous carrier is converted into a coloured reaction product in the control section (3).

8. Immunoassay apparatus according to Claim 1 to 7, **characterised in that** the apparatus is contained in a case (6) which protects against environmental influences.

9. Immunoassay apparatus according to Claim 8, **characterised in that** the case is made of a plastics material.

10. Immunoassay apparatus according to Claim 9, **characterised in that** the case (6) is composed of two parts.

## Revendications

1. Dispositif d'essai immunologique constitué d'un support poreux sec comportant au moins deux zones (1,2) reliées, lorsqu'elles sont à l'état mouillé, par capillarité l'une à l'autre, la première zone (1) contenant un révélateur marqué, adapté à la substance à détecter, qui se présente sous forme d'un anticorps approprié ou d'un antigène approprié, et la deuxième zone (2) contenant un réactif immobilisé permettant de fixer la substance à détecter, et qui se présente sous forme d'un antigène ou anticorps correspondant, dans lequel le produit résultant de la réaction entre la substance à détecter et le révélateur marqué migre à l'état mouillé, par capillarité, de la première zone dans la deuxième zone, **caractérisé par le fait que** le réactif révélateur marqué est un réactif coloré capable de réagir avec la nicotine ou la cotinine.

2. Dispositif d'essai immunologique selon la revendication 1, **caractérisé par le fait que** le réactif immobilisé permettant de fixer la substance à détecter est un antigène de type nicotine ou cotinine.

3. Dispositif d'essai immunologique selon la revendication 1, **caractérisé par le fait que** le réactif immobilisé permettant de fixer la substance à détecter est un anticorps anti-nicotine ou anti-cotinine.

4. Dispositif d'essai immunologique selon l'une des revendications 1 à 3, **caractérisé par le fait que** le produit migrant, résultant de la réaction entre la substance à détecter et le révélateur marqué, est couplé à des particules de polymères colorées ou à des sols métalliques.

5. Dispositif d'essai immunologique selon la revendication 4, **caractérisé par le fait que** le conjugué contient un sol à base d'or.

6. Dispositif d'essai immunologique selon l'une des revendications 1 à 5, **caractérisé par le fait que** la deuxième zone (2) est suivie d'une zone de contrôle (3) servant à la vérification du bon déroulement de l'essai.

7. Dispositif d'essai immunologique selon la revendication 6, **caractérisé par le fait que** l'antigène marqué ou l'anticorps marqué ou un véhicule aqueux sont convertis dans la zone de contrôle (3) en un produit réactionnel coloré.

8. Dispositif d'essai immunologique selon une des revendications 1 à 7, **caractérisé par le fait que** le dispositif est conservé dans une enveloppe (6) qui le protège contre les influences de l'environnement.

9. Dispositif d'essai immunologique selon la revendication 8, **caractérisé par le fait que** l'enveloppe est en matière plastique.

10. Dispositif d'essai immunologique selon la revendication 9, **caractérisé par le fait que** l'enveloppe (6) est une enveloppe en deux parties.
